(19) Europäisches Patentamt
European Patent Office
Office européen des brevets

(11) Veröffentlichungsnummer : **0 364 875 B1**

(12) # EUROPÄISCHE PATENTSCHRIFT

(45) Veröffentlichungstag der Patentschrift : **24.03.93 Patentblatt 93/12**

(51) Int. Cl.$^5$ : **C07F 9/6561**

(21) Anmeldenummer : **89118924.3**

(22) Anmeldetag : **12.10.89**

(54) **Riboflavin-4'5'-cyclo-phosphorsäureesterchlorid, dessen Herstellung und Verwendung zur Herstellung von Riboflavin-5'-phosphat (5'-FMN) bzw. dessen Natriumsalz.**

(30) Priorität : **19.10.88 DE 3835563**

(43) Veröffentlichungstag der Anmeldung : **25.04.90 Patentblatt 90/17**

(45) Bekanntmachung des Hinweises auf die Patenterteilung : **24.03.93 Patentblatt 93/12**

(84) Benannte Vertragsstaaten : **CH DE FR GB IT LI NL**

(56) Entgegenhaltungen :
**CHEMICAL ABSTRACTS, Band 105, Nr. 9, 1. September 1986, Seite 627, Zusammenfassung Nr. 78740x, Columbus, Ohio, USA; B ZAGALAK et al.: "Phosphorylation of unprotected D-neopterin" & Biochem. Clin. Aspects Pteridines 1985, Band 4 (Cancer, Immunol., Metab. Dis.), Seiten 159-167**
**CHEMICAL ABSTRACTS, Band 88, Nr.19, 8. Mai 1978, Seite 501, Zusammenfassung Nr.136229f, Columbus, Ohio, USA; V K SIDORENKO et al.: "Synthesis of some gamma-alkoxy-alpha, beta-propylene glycol esters of phosphoric acid" & Dokl. Akad. Nauk. Tadzh. SSR 1977, Band 20, Nr. 11, Seiten 22-24**

(73) Patentinhaber : **BASF Aktiengesellschaft**
**Carl-Bosch-Strasse 38**
**W-6700 Ludwigshafen (DE)**

(72) Erfinder : **Grimmer, Johannes**
**Duererstrasse 12**
**W-6700 Ludwigshafen (DE)**
Erfinder : **Kiefer, Hans, Dr.**
**Im Sandgarten 5**
**W-6706 Wachenheim (DE)**

**EP 0 364 875 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Riboflavin-4′,5′-cyclo-phosphorsäureesterchlorid der Formel I

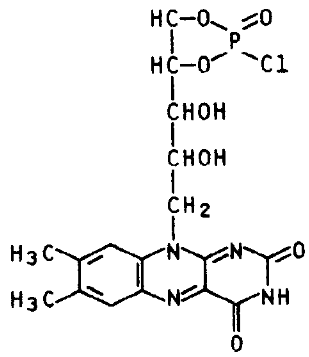

(I),

ein Verfahren zu dessen Herstellung sowie dessen Verwendung zur Herstellung von Riboflavin-5′-phosphat (5′-Flavinmononucleotid und daher nachfolgend als 5′-FMN bezeichnet) bzw. zur Herstellung des handelsüblichen Mononatriumsalzes von 5′-FMN.

5′-FMN ist eine Verbindung, die eine wesentliche Rolle als Coenzym in verschiedenen Enzymreaktionen im lebenden Körper spielt und die deshalb in Form ihrer Salze, insbesondere in Form von Natrium-5′-FMN, als Zusatz für Arzneimittel, Nahrungsmittel und Futterstoffe verwendet wird. Natrium-5′-FMN dient auch als Ausgangsmaterial für Flavin-adenin-dinukleotid, das als therapeutisches Mittel gegen Vitamin-$B_2$-Mangel eingesetzt wird.

Industriell wird Natrium-5′-FMN im allgemeinen durch direktes Umsetzen von Riboflavin mit einem Phosphorylierungsmittel, wie partiell hydrolysiertem Phosphoroxychlorid, und anschließendes Behandeln des erhaltenen 5′-FMN mit Natronlauge gewonnen. Die selektive Phosphorylierung von Riboflavin ist nicht ganz einfach. So arbeitet man beispielsweise gemäß US-2 610 177 mit einem großen Überschuß an Phosphoroxichlorid. Gemäß C.A. 83 (1975), 79551a, C.A. 83 (1975), 79549f (JA-OS 50/25 597) bzw. C.A. 83 (1975), 79550z (JA-OS 50/25 598) wurde ein geringer Überschuß an Phosphoroxichlorid in Lösungsmitteln, wie Tetrahydrofuran, Diethylenglykoldimethylether, Monoethylenglykoldimethylether, Triethylphosphat, 1,2-Dichlor- oder 1,2-Dibromethan empfohlen. Bei der Nacharbeitung der Beispiele ergab sich, daß unter den angegebenen Bedingungen in vielen Fällen überhaupt kein 5′-FMN, in anderen Fällen nur äußerst geringe Mengen an 5′-FMN erhalten werden konnten. Die in loc. cit. angegebenen hohen Ausbeuten beruhen vermutlich auf Problemen bei der Analytik.

In einigen Fällen wird in Gegenwart von Pyridin (vgl. US 2 111 491) oder in Gegenwart von Acetonitril (vgl. Techn. Rapport Nr. 2715 (1979) von Frantz Kaufmann der Grindstedt Verket, Dänemark) phosphoryliert.

Bei allen bekannten Herstellungsverfahren wird zunächst ein Rohprodukt erhalten, das noch erhebliche Mengen an unumgesetztem Riboflavin, sowie isomere Mono- und Polyphosphate als Nebenprodukte enthält. Daher muß das 5′-FMN einer technisch aufwendigen Reinigungsprozedur unterzogen werden, damit Produkte erhalten werden, die den Reinheitskriterien der US- und EP-Pharmakopoen entsprechen. So ist z.B. aus Chemical Engineering, Nov. 1954, Seiten 120ff bekannt, daß in einem Produktionsprozess das 5′-FMN angereichert wird, indem man das Isomerengemisch durch mehrmalige Behandlung mit Ethanolamin in Form von Monoammoniumsalzen in Lösung bringt und von nicht umgesetztem und ungelöstem Riboflavin abtrennt.

Allein an den aufwendigen Verfahrensschritten und darüber hinaus dem Einsatz von großen Mengen Phosphoroxichlorid in Bezug auf das zu phosphorylierende Riboflavin (Vitamin $B_2$) ist erkennbar, daß solche Verfahren einen nicht unerheblichen Einfluß auf die Chloridbelastung im Abwasser darstellen können. Auch bei Reinigungsverfahren für Vitamin-$B_2$-Phosphat durch Absorption an einem Celluloseionenaustauscher und Elution mit einem Natriumoxalat/Oxalsäure bzw. Ammoniumformiat/Ameisensäurepuffer (vgl. JA-AS 47/8836 und JA-AS 47/8554) wird das Verfahren nicht wirtschaftlicher und umweltfreundlicher, da bei technischer Nutzung zu große Mengen an Puffersalzen zur Anwendung kommen.

Es wurde nun gefunden, daß man überraschenderweise Salze des 5′-FMN besonders vorteilhaft erhält, wenn man nicht, wie nach dem Stand der Technik, das freie Riboflavin zur Phosphorylierung, sondern dessen Metallsalze, vorzugsweise die Alkalisalze, insbesondere das Kaliumsalz (II) des Riboflavins einsetzt. Hierbei bildet sich primär das unseres Wissens bisher in der Literatur nicht beschriebene Riboflavin-4′,5′-cyclo-phosphorsäureesterchlorid der Formel I, das in kristalliner Form abgetrennt werden kann. Letzteres kann dann unter geeigneten Bedingungen unter Ringöffnung hydrolysiert und durch Teilneutralisation mit Natronlauge bei pH

5,5 in das Natriumsalz von 5′-FMN überführt werden.

Die Umsetzung erfolgt bei Verwendung des Kaliumsalzes nach folgender Gleichung:

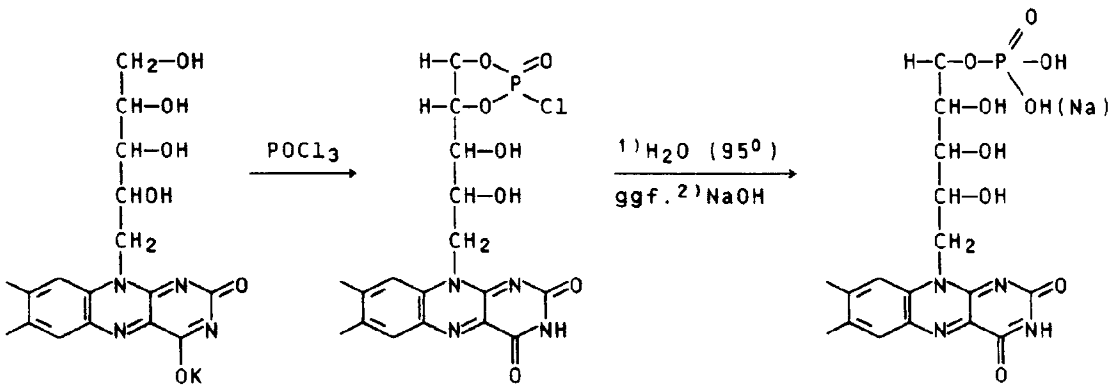


Es war sehr überraschend, daß bei der Umsetzung der Alkalimetallsalze des Riboflavins mit Phosphoroxichlorid oder mit einem Ester des Phosphoroxidichlorids der Angriff des phosphorylierenden Agens in 4′-5′-Stellung des Ribitylrestes erfolgt. Aus der Literatur ist bekannt, daß die negative Ladung im Anion des Riboflavins in den Heterocyclen lokalisiert ist, so daß jeder Fachmann den Angriff des Phorphorylierungsagenses an der Stelle mit der höchsten Ladungsdichte in Pos. 4 und 5 im Isoalloxazinring vermutet hätte und nicht an der weitentfernten 4′,5′-Position des Ribitylrests.

Gegenstand der Erfindung ist daher neben dem Riboflavin-4′,5′-cyclo-phosphorsäureesterchlorid der Formel I ein Verfahren zu dessen Herstellung, das dadurch gekennzeichnet ist, daß man ein Alkalisalz, insbesondere das Kaliumsalz des Riboflavins in einem geeigneten aprotischen Lösungsmittels bei Temperaturen von 20 bis 50, vorzugsweise bei etwa 30 bis 45°C mit 1,2 bis 3 Mol Phosphoroxichlorid pro Mol des Alkalisalzes umsetzt und gegebenenfalls das aus dem Reaktionsgemisch auskristallisierende Produkt durch Filtration isoliert.

Als aprotische Lösungsmittel sind für die Umsetzung insbesondere lineare oder cyclische Ether, wie Monoethylenglykoldimethylether, Diethylenglykoldimethylether, Triethylenglykoldimethylether, Tetrahydrofuran oder Dioxan geeignet.

Das als Ausgangsverbindung verwendete Alkalisalz wird auf einfache Weise dadurch erhalten, daß man das Riboflavin in äquimolaren Mengen einer verdünnten wäßrigen Alkalihydroxidlösung löst und das erhaltene Salz durch Zutropfen von Methanol zur Kristallisation bringt. Durch Filtration, Waschen mit Methanol und Trocknen erhält man das Alkalisalz in nahezu quantitativer Ausbeute. Das Alkalisalz enthält in getrocknetem Zustand ein Kristallwasser und ist so stabil wie Riboflavin selbst.

Mit besonderem Vorteil arbeitet man bei der Herstellung des Phosphorsäureesterchlorids der Formel I so, daß man das Riboflavin-Alkalisalz in eine Lösung von etwa 2,8 Mol Phosphoroxichlorid pro Mol Alkalisalz (d.h. einen ca. 1,8 molaren Überschuß) in einem geeigneten aprotischen Lösungsmittel einträgt. Nach 2stündiger Reaktion bei 40 bis 45°C ist bereits eine mehr als 95 %ige Umsetzung erreicht. Das gebildete kristalline Riboflavin-4′,5′-cyclo-phosphorsäureesterchlorid kann durch Filtration isoliert werden, oder aber gleich durch Zugabe von Wasser zum Reaktionsgemisch und Erhitzen zum gewünschten 5′-FMN hydrolysiert und isomerisiert werden. Aus dem erhaltenen 5′-FMN kann dann gewünschtenfalls durch Teilneutralisation des Mononatriumsalz von 5′-FMN erhalten werden.

Eine Zwischenisolierung des 5′-FMN ist meistens nicht nötig, da man in einem Verfahrensschritt durch Teilneutralisation der freien Säure das Natriumsalz des 5′-FMN in gewünschter Reinheit erhält.

Gegenstand der Erfindung ist dementsprechend auch die Verwendung von Riboflavin-4′,5′-cyclo-phosphorsäureesterchlorid der Formel I zur Herstellung von 5′-FMN durch Hydrolysieren und Isomerisieren sowie zur Herstellung des Mononatriumsalzes von 5′-FMN durch Hydrolysieren, Isomerisieren und Teilneutralisation.

Gegenstand der Erfindung ist weiterhin das oben beschriebene Verfahren, das dadurch gekennzeichnet ist, daß man zur Herstellung von 5′-FMN bzw. von dessen Natriumsalz das erhaltene Riboflavin-4′,5′-cyclophosphorsäurechlorid der Formel I

a) zum Riboflavin-4′,5′-phosphorsäureester hydrolysiert und

b) diesen zu 5′-FMN isomerisiert und

c) dieses gewünschtenfalls mit Natriumhydroxid zu dem Mononatriumsalz von 5′-FMN umsetzt.

Zur Durchführung dieses Verfahrens geht man im allgemeinen so vor, daß man das Riboflavin-4′,5′-phosphorsäureesterchlorid der Formel I enthaltende Reaktionsgemisch

a) rasch mit 30 bis 50, vorzugsweise 32 bis 35 Mol Wasser pro Mol Phosphorsäureesterchlorid versetzt, wobei die Temperatur auf über 90°C ansteigt und durch Hydrolyse Riboflavin-4′,5′-phosphorsäureester gebildet wird,

b) das Reaktionsgemisch durch Einleiten von Dampf noch 5 bis 15, vorzugsweise 8 bis 12 Minuten auf Temperaturen von 80 bis 100°C, vorzugsweise 85 bis 90°C hält, wobei der gebildete Riboflavin-4′,5′-phosphorsäureester im wesentlichen zu 5′-FMN isomerisiert wird,

c) durch Zugabe von 68 bis 100 Mol Wasser zu dem Reaktionsgemisch und die dadurch bedingte Abkühlung die Isomerisierung abbricht und

d) ggf. zur Herstellung des Mononatriumsalzes von 5′-FMN das Reaktionsgemisch mit Natriumhydroxid auf einen pH-Wert von 5,5 bis 6 einstellt.

Geht man von isoliertem Riboflavin-4′,5′-cyclophosphorsäurechlorid aus, so muß man

a) dieses in eine zur Lösung ausreichende Menge von auf 80 bis 95°C erwärmten Wassers eintragen,

b) das Reaktionsgemisch durch Einleiten von Dampf noch 5 bis 15 Minuten auf Temperaturen von 80 bis 100°C halten,

c) durch anschließende Zugabe von 68 bis 100 Mol Wasser die Isomerisierung abbrechen und

d) gewünschtenfalls zur Herstellung des Mononatriumsalzes von 5′-FMN das Reaktionsgemisch mit Natriumhydroxid auf einen pH-Wert von 5,5 bis 6 einstellen.

Bei der erfindungsgemäßen Herstellung von 5′-FMN bzw. dessen Mononatriumsalz ist darauf zu achten, daß das Riboflavin-4′,5′-cyclophosphorsäurechlorid enthaltende Reaktionsgemisch möglichst schnell auf Temperaturen von 80 bis 100°C gelangt und die Temperatur für die angegebene Zeit ohne zwischenzeitliches Absinken der Temperatur bei 80 bis 100°C gehalten wird, da sonst ein Produkt mit unakzeptabel hohem Riboflavingehalt erhalten wird.

Die Umsetzung des Riboflavin-5′-phosphats mit NaOH zu seinem Mononatriumsalz erfolgt im allgemeinen bei Temperaturen von 20 bis 50°C, vorzugsweise 30 bis 40°C.

Gegenstand der Erfindung ist auch das sich insgesamt ergebende elegante Eintopfverfahren zur Herstellung von reinem Riboflavin-5′-phosphat bzw. von seinem Mononatriumsalz, das dadurch gekennzeichnet ist, daß man

A) ein Alkalisalz des Riboflavins in einem geeigneten aprotischen Lösungsmittel bei Temperaturen von 20 bis 50°C mit 1,2 bis 3 Mol Phosphoroxichlorid pro Mol des Alkalisalzes umsetzt,

B) das so erhaltene, das neue Riboflavin-4′,5′-cyclo-phosphorsäureesterchlorid der Formel I enthaltende Reaktionsgemisch rasch mit 30 bis 50 Mol Wasser pro Mol Esterchlorid versetzt, wobei die Temperatur auf über 90°C ansteigt,

C) das Reaktionsgemisch durch Einleiten von Dampf noch 5 bis 15 Minuten auf Temperaturen von 80 bis 100°C hält,

D) anschließend 68 bis 100 Mol Wasser zu dem Reaktionsgemisch addiert und das auskristallisierende Riboflavin-5′-phosphat isoliert oder gewünschtenfalls

E) das gemäß D) erhaltene Reaktionsgemisch bei Temperaturen von 20 bis 50°C, vorzugsweise 30 bis 40°C mit NaOH auf einen pH-Wert zwischen 5,5 und 6 einstellt und das auskristallisierende Mononatriumsalz von Riboflavin-5′-phosphat isoliert.

Das bei dem erfindungsgemäßen Verfahren erhaltene Riboflavin-5′-phosphat enthält im allgemeinen weniger als 6 % Riboflavin und 75 bis 80 % Riboflavin-5′-phosphat und entspricht damit den auf pharmazeutischem Gebiet geltenden Reinheitsanforderungen. Anschließende aufwendige Reinigungsoperationen erübrigen sich.

Das Riboflavin-4′,5′-cyclo-phosphorsäureesterchlorid der Formel I ist ein auf einfache Weise erhältliches Zwischenprodukt, das einen einfachen Weg zu dem begehrten 5′-FMN und dessen Mononatriumsalz in großer Reinheit eröffnet.

Beispiel 1

Herstellung von Riboflavin-4′,5′-cyclo-phosphorsäureesterchlorid

| | |
|---|---|
| In 180 ml | Diethylenglykoldimethylether wurden |
| 60,12 g | (0,392 mol) Phosphoroxichlorid eingetragen und hierzu unter Rühren |
| 60 g | (0,139 mol) Riboflavin-Kalium-Salz, fein pulverisiert, portionsweise zugegeben, wobei die Temperatur auf 30°C anstieg. Anschließend wurde das Reaktionsgemisch auf 45°C erwärmt und 2 Stunden (h) bei 45°C gerührt. Nach Abkühlen auf Raumtemperatur (RT) wurde die Suspension unter Stickstoff abgesaugt, dann mit Diethylenglykoldimethylether und anschließend mit Aceton gewaschen und schließlich unter vermindertem Druck getrocknet. |

Die Ausbeute betrug 62,0 g entsprechend 97,8 % der Theorie.

Analytik:

- Gehalt nach HPLC an cyclischem Chlorid: 95 %
- Das Molekülion wurde mittels FAB -MS-Methode (vgl. K.L. Rinehart in Science 218 (1982) Seite 254) gemessen.
- Die Molekulargewichtsbestimmung ergab ein Molekulargewicht von 456 g/Mol, welches der Titelverbindung entspricht.

Das Produkt enthielt noch Kalium-Salze.

Beispiel 2

Herstellung von Riboflavin-5′-phosphat

In ein Gemisch von

| | |
|---|---|
| 180 ml | Diethylenglykoldimethylether und |
| 60,12 g | (0,392 mol) = 36 ml Phosphoroxichlorid wurden |
| 60 g | (0,139 mol) Riboflavin-Kalium-Salz, fein pulverisiert, portionsweise eingetragen und danach das Reaktionsgemisch 2 h bei 45°C gerührt. Anschließend wurden bei dieser Temperatur rasch |
| 75 g | Wasser zugegeben, wobei die Temperatur schnell auf 90 bis 95°C anstieg. Durch Einblasen von Dampf wurde die Temperatur 10 bis 15 Minuten bei 90 bis 95°C gehalten. Während des anschließenden Zutropfens von |
| 170 ml | Wasser wurde das Reaktionsgemisch langsam abgekühlt, wobei schon zwischen 70 und 80°C Riboflavin-5′-phosphat auszufallen begann. Nach 2 h Nachrührzeit bei 20 bis 25°C wurde abgesaugt und der Rückstand zuerst mit einem Wasser/Ethanol-Gemisch (je 50 Vol.%), danach mit wenig reinem Ethanol gewaschen und anschließend bei 75°C unter vermindertem Druck getrocknet. |

Die Ausbeute betrug ca. 52 g entsprechend 82,0 % der Theorie.

Analytik:

Nach HPLC enthält das Produkt ca. 75 bis 78 % Riboflavin-5'-phosphat;
ca. 9 bis 11 % Riboflavin-4'-phosphat;
ca. 5 bis 7 % Riboflavin-3'-phosphat
und ca. 4 bis 6 % freies Riboflavin.

Beispiel 3

Herstellung des Natriumsalzes von Riboflavin-5′-phosphat

Man arbeitete zunächst wie in Beispiel 2 beschrieben, kühlte das Reaktionsgemisch nach der Hydrolyse und Zutropfen von 170 ml Wasser auf 30°C ab und stellte bei Temperaturen zwischen 30 und 40°C durch langsames Zudosieren einer 25 %igen wäßrigen Natronlauge einen pH-Wert von ca. 5,5 ein. Nach Erreichen des pH-Wertes wurde das Reaktionsgemisch auf 20°C abgekühlt, dann sofort abgesaugt und nach Waschen mit einem Wasser-/Ethanol-Gemisch (je 50 Vol.%) und Ethanol unter vermindertem Druck bei 75°C getrocknet.

Die Ausbeute betrug 54,5 g entsprechend 82,1 % der Theorie.

Analytik

| | | | |
|---|---|---|---|
| Nach HPLC enthält das Produkt | | 9 bis 11 % | Riboflavin-4'-phosphat-Natrium, |
| | | 75 bis 78 % | Riboflavin-5'-phosphat-Natrium |
| | und | 5 bis 6 % | unumgesetztes Riboflavin |
| spezifische Drehung | | : +37,3 bis +38° | |
| Gehalt an Natrium | | : ca. 5 % | |
| pH-Wert einer 3 %igen wäßrigen Lösung: | | 5 bis 6,3. | |

Beispiel 4

A.
In 100 ml Diethylenglykoldimethylether wurden 12 ml Phosphoroxichlorid eingetragen und hierzu unter Rühren 20 g (0,048 mol) Riboflavin-Kalium-Salz feinpulverisiert, portionsweise zugegeben, wobei die Temperatur auf 30°C anstieg. Anschließend wurde das Reaktionsgemisch auf 35°C erwärmt und 3 h bei 35°C gerührt. Nach Abkühlen auf RT wurde die Suspension unter $N_2$ abgesaugt, dann mit 100 ml Diethylenglykoldimethylether gewaschen und getrocknet. Nach HPLC-Analyse bestand das Produkt zu 91 % aus Riboflavin-4',5'-phosphorsäurechlorid und zu nur 1 % aus unumgesetztem Riboflavin.
B.
200 ml Wasser wurden auf 75 bis 85°C erhitzt und bei dieser Temperatur das unter A. erhaltene Produkt portionsweise eingetragen. Anschließend wurde das Reaktionsgemisch noch 15 min. bei Temperaturen von 90 bis 95°C gerührt. Dann wurde das Reaktionsgemisch langsam abgekühlt und bei 40°C mit 25 %iger wäßriger Natronlauge auf pH 5,5 eingestellt. Nach Erreichen des pH-Wertes wurde das Reaktionsgemisch auf 20°C abgekühlt, dann sofort abgesaugt und nach Waschen mit einem Wasser-Ethanol-Gemisch (je 50 Vol.%) und Ethanol unter vermindertem Druck bei 75°C getrocknet. Die Ausbeute betrug 19 g.

| | | | |
|---|---|---|---|
| Analytik: Nach HPLC enthält das Produkt | | ca. 76 % | Riboflavin-5'-phosphat, |
| | | ca. 9 % | Riboflavin-4'-phosphat |
| | und | ca. 5 % | Riboflavin. |

## Patentansprüche

1. Riboflavin-4',5'-cyclo-phosphorsäureesterchlorid der Formel I

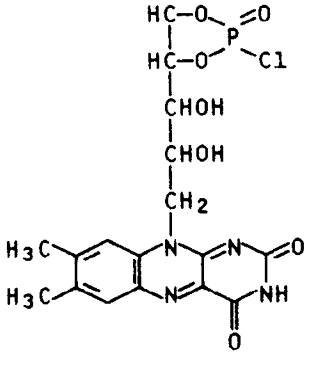

(I),

2. Verfahren zur Herstellung von Riboflavin-4',5'-cyclo-phosphorsäureesterchlorid der Formel I gemäß Anspruch 1, dadurch gekennzeichnet, daß man ein Alkalisalz des Riboflavins in einem geeigneten aprotischen Lösungsmittel bei Temperaturen von 20 bis 50°C mit 1,2 bis 3 Mol Phosphoroxichlorid pro Mol des Alkalisalzes umsetzt und gegebenenfalls das aus dem Reaktionsgemisch auskristallisierende Produkt

durch Filtration isoliert.

3. Verfahren gemäß Anspruch 2 zur Herstellung von Riboflavin-4′,5′-cyclo-phosphorsäureesterchlorid der Formel I, dadurch gekennzeichnet, daß man die Umsetzung des Alkalisalzes von Riboflavin mit Phosphoroxichlorid bei Temperaturen von 30 bis 45°C durchführt.

4. Verfahren gemäß Anspruch 2 zur Herstellung von Riboflavin-4′,5′-cyclo-phosphorsäureesterchlorid der Formel I, dadurch gekennzeichnet, daß man als aprotisches Lösungsmittel Monoethylenglykoldimethylether, Diethylenglykoldimethylether, Triethylenglykoldimethylether, Tetrahydrofuran oder Dioxan verwendet.

5. Verwendung von Riboflavin-4′5′-cyclo-phosphorsäureesterchlorid der Formel I gemäß Anspruch 1 zur Herstellung von Riboflavin-5′-phosphat bzw. dessen Mononatriumsalz durch Hydrolysieren, Isomerisieren und gewünschtenfalls Teilneutralisation.

6. Verfahren gemäß Anspruch 5, dadurch gekennzeichnet, daß man zur Herstellung von Riboflavin-5′-phosphat bzw. seinem Mononatriumsalz das erhaltene Riboflavin-4′,5′-cyclo-phosphorsäurechlorid der Formel I
   a) zum Riboflavin-4′,5′-phosphorsäureester hydrolysiert, diesen
   b) zu Riboflavin-5′-phosphat isomerisiert und dieses
   c) gewünschtenfalls mit Natriumhydroxid zu seinem Mononatriumsalz umsetzt.

7. Verfahren gemäß Anspruch 5, dadurch gekennzeichnet, daß man zur Herstellung von Riboflavin-5′-phosphat das erhaltene, Riboflavin-4′,5′-phosphorsäurechlorid der Formel I enthaltende Reaktionsgemisch
   a) rasch mit 30 bis 50 Mol Wasser pro Mol Riboflavin-4,5′-cyclo-phosphorsäureesterchlorid versetzt, wobei die Temperatur auf über 90°C ansteigt und durch Hydrolyse Riboflavin-4′,5′-phosphorsäureester gebildet wird,
   b) das Reaktionsgemisch durch Einleiten von Dampf noch 5 bis 15 Minuten auf Temperaturen von 80 bis 100°C hält, wobei der gebildete Riboflavin-4′,5′-phosphorsäureester zu Riboflavin-5′-phosphat isomerisiert wird, und
   c) durch anschließende Zugabe von 68 bis 100 Mol Wasser zu dem Reaktionsgemisch und die dadurch bedingte Abkühlung die Isomerisierungsreaktion abbricht.

8. Verfahren gemäß Anspruch 5, dadurch gekennzeichnet, daß man zur Herstellung des Mononatriumsalzes von Riboflavin-5′-phosphat das erhaltene, das Riboflavin-4′,5′-phosphorsäureesterchlorid der Formel I enthaltende Reaktionsgemisch
   a) rasch mit 30 bis 35 Mol Wasser pro Mol Riboflavin-4′,5′-cyclo-phosphorsäureesterchlorid versetzt, wobei die Temperatur auf über 90°C ansteigt und durch Hydrolyse Riboflavin-4′,5′-phosphorsäureester gebildet wird,
   b) das Reaktionsgemisch durch Einleiten von Dampf noch etwa 5 bis 15 Minuten auf Temperaturen von 80 bis 100°C hält, wobei der gebildete Riboflavin-4′,5′-phosphorsäureester zu Riboflavin-5′-phosphat isomerisiert wird,
   c) durch anschließende Zugabe von 68 bis 100 Mol Wasser zu dem Reaktionsgemisch und die dadurch bedingte Abkühlung die Isomerisierungsreaktion abbricht und
   d) das Reaktionsgemisch mit Natriumhydroxid auf einen pH-Wert von 5,5 bis 6 einstellt.

9. Verfahren zur Herstellung von Riboflavin-5′-phosphat bzw. dessen Mononatriumsalz, dadurch gekennzeichnet, daß man
   a) kristallines Riboflavin-4′,5′-cyclo-phosphorsäureesterchlorid in eine zu seiner Lösung ausreichende Menge von auf 80 bis 95°C erwärmten Wassers einträgt,
   b) das Reaktionsgemisch durch Einleiten von Dampf noch 5 bis 15 Minuten auf Temperaturen von 80 bis 100°C hält,
   c) anschließend von 68 bis 100 Mol Wasser zugibt und das auskristallisierende Riboflavin-5′-phosphat isoliert oder gewünschtenfalls
   d) zur Herstellung des Mononatriumsalzes das Reaktionsgemisch mit Natriumhydroxid auf einen pH-Wert von 5,5 bis 6 einstellt und das auskristallisierende Mononatriumsalz des Riboflavin-5′-phosphats isoliert.

10. Verfahren zur Herstellung von reinem Riboflavin-5′-phosphat bzw. von seinem Mononatriumsalz, dadurch

gekennzeichnet, daß man

A) ein Alkalisalz des Riboflavins in einem geeigneten aprotischen Lösungsmittel bei Temperaturen von 20 bis 50°C mit 1,2 bis 3 Mol Phosphoroxichlorid pro Mol des Alkalisalzes umsetzt,

B) das so erhaltene, das neue Riboflavin-4',5'-cyclo-phosphorsäureesterchlorid der Formel I enthaltende Reaktionsgemisch rasch mit 30 bis 50 Mol Wasser pro Mol Esterchlorid versetzt, wobei die Temperatur auf über 90°C ansteigt,

C) das Reaktionsgemisch durch Einleiten von Dampf noch 5 bis 15 Minuten auf Temperaturen von 80 bis 100°C hält,

D) anschließend 68 bis 100 Mol Wasser zu dem Reaktionsgemisch addiert und das auskristallisierende Riboflavin-5'-phosphat isoliert oder gewünschtenfalls

E) das gemäß D) erhaltene Reaktionsgemisch bei Temperaturen von 20 bis 50°C mit NaOH auf einen pH-Wert zwischen 5,5 und 6 einstellt und das auskristallisierende Mononatriumsalz von Riboflavin-5'-phosphat isoliert.

## Claims

1. Riboflavin-4', 5'-cyclophosphoric acid ester chloride of the formula I

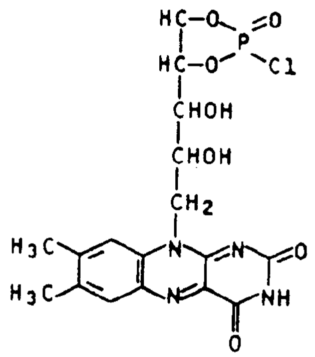

(I)

2. A process for the preparation of riboflavin -4', 5'-cyclophosphoric acid ester chloride of the formula I as claimed in claim 1, which comprises reacting an alkali metal salt of riboflavin in a suitable aprotic solvent at from 20 to 50°C with from 1.2 to 3 moles of phosphorous oxychloride per mole of the salt and, if required, isolating the product which crystallizes out of the reaction mixture by filtration.

3. A process as claimed in claim 2 for the preparation of riboflavin-4',5'-cyclophosphoric acid ester chloride of the formula I, wherein the reaction of the alkali metal salt of riboflavin with phosphorus oxychloride is carried out at from 30 to 45°C.

4. A process as claimed in claim 2 for the preparation of riboflavin-4', 5'-cyclophosphoric acid ester chloride of the formula I, wherein monoethylene glycol dimethyl ether, diethylene glycol dimethyl ether, triethylene glycol dimethyl ether, tetrahydrofuran or dioxane is used as aprotic solvent.

5. The use of riboflavin-4',5'-cyclophosphoric acid ester chloride of the formula I as claimed in claim 1 for the preparation of riboflavin 5'-phosphate or its monosodium salt by hydrolysis, isomerization and, if required, partial neutralization.

6. A process is claimed in claim 5, wherein, for the preparation of riboflavin 5'-phosphate, or of its monosodium salt, the riboflavin-4',5'-cyclophosphoric acid ester chloride of the formula I

a) is hydrolyzed to give riboflavin-4',5'-phosphoric acid ester, which

b) is isomerized to give riboflavin 5'-phosphate and this

c) is reacted, if desired, with sodium hydroxide to give its monosodium salt.

7. A process as claimed in claim 5, wherein for the preparation of riboflavin 5'-phosphate to the reaction mixture containing the riboflavin-4',5'-phosphoric acid ester chloride of the formula I

a) there are rapidly added from 30 to 50 moles of water per mole of riboflavin-4',5'-cyclophosphoric acid ester chloride, in the course of which the temperature rises to above 90°C and riboflavin-4',5'-phosphoric acid ester is formed by hydrolysis,

b) the reaction mixture is kept for a further 5-15 minutes at from 80 to 100°C by introducing steam, in the course of which the riboflavin-4′,5′-phosphoric acid ester formed is isomerized to riboflavin 5′-phosphate, and
c) the isomerization is interrupted by addition of 68-100 moles of water to the reaction mixture and by the cooling which this causes.

8. A process as claimed in claim 5, wherein for the preparation of the monosodium salt of riboflavin 5′-phosphate to the reaction mixture containing the riboflavin-4′,5′-phosphoric acid ester chloride of the formula I
a) there are rapidly added from 30 to 35 moles of water per mole of riboflavin-4′,5′-cyclophosphoric acid ester chloride, in the course of which the temperature rises to above 90°C and riboflavin-4′,5′-phosphoric acid ester is formed by hydrolysis,
b) the reaction mixture is kept for a further 5-15 minutes at from 80 to 100°C by introducing steam, in the course of which the riboflavin-4′, 5′-phosphoric acid ester formed is isomerized to riboflavin 5′-phosphate,
c) the isomerization is interrupted by addition of 68-100 moles of water to the reaction mixture and by the cooling which this causes, and
d) the reaction mixture is brought to a pH of from 5.5 to 6 by means of sodium hydroxide.

9. A process for the preparation of riboflavin 5′-phosphate or its monosodium salt, wherein
a) crystalline riboflavin-4′, 5′-cyclophosphoric acid ester chloride is introduced into an amount of water, heated to 80-95°C, which suffices to effect dissolution of the product,
b) the reaction mixture is kept for a further 5-15 minutes at 80-100°C by introducing steam,
c) thereafter 68-100 moles of water are added and the riboflavin 5′-phosphate which crystallizes out is isolated, or, if desired,
d) to prepare the monosodium salt, the reaction mixture is brought to a pH of 5.5-6 with sodium hydroxide and the monosodium salt of the riboflavin 5′-phosphate, which crystallizes out, is isolated.

10. A process for the preparation of pure riboflavin 5′-phosphate or of its monosodium salt, wherein
A) an alkali metal salt of riboflavin in a suitable aprotic solvent is reacted, at from 20 to 50°C, with from 1.2 to 3 moles of phosphorous oxychloride per mole of the alkali metal salt,
B) to the reaction mixture thus obtained, which contains the novel riboflavin-4′, 5′-cyclophosphoric acid ester chloride of the formula I, there are rapidly added from 30 to 50 moles of water per mole of ester chloride, in the course of which the temperature rises to above 90°C,
C) the reaction mixture is kept at from 80 to 100°C for a further 5-15 minutes by introducing steam,
D) thereafter from 68 to 100 moles of water are added to the reaction mixture and the riboflavin 5′-phosphate which crystallizes out is isolated, or, if desired,
E) the reaction mixture obtained according to D) is brought, at from 20 to 50°C, to a pH of from 5.5 to 6 by means of NaOH and the monosodium salt of riboflavin 5′-phosphate which crystallizes out, is isolated.

## Revendications

1. Chlorure de riboflavine - 4′,5′-cyclophosphate de formule I

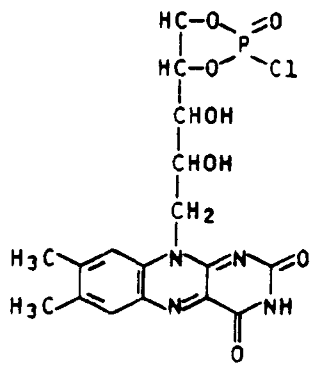

(I),

2. Procédé de préparation de chlorure de riboflavine -4′,5′-cyclophosphate de formule I selon la revendication 1, caractérisé en ce qu'on fait réagir un sel alcalin de la riboflavine dans un solvant aprotique approprié, à des températures de 20 à 50°C, avec 1,2 à 3 moles d'oxychlorure de phosphore par mole du sel alcalin, et on isole éventuellement par filtration le produit qui se sépare a l'état cristallin du mélange réactionnel.

3. Procédé selon la revendication 2 pour la préparation de chlorure de riboflavine-4′,5′-cyclophosphate de formule I, caractérisé en ce qu'on conduit la réaction du sel alcalin de riboflavine avec l'oxychlorure de phosphore à des températures de 30 à 45°C.

4. Procédé selon la revendication 2 pour la préparation de chlorure de riboflavine -4′,5′-cyclophosphate de formule I, caractérisé en ce qu'on utilise, comme solvant aprotique, de l'éther diméthylique de monoéthylèneglycol, de l'éther diméthylique de diéthylèneglycol, de l'éther diméthylique de triéthylèneglycol, du tétrahydrofuranne ou du dioxanne.

5. Utilisation de chlorure de riboflavine-4′,5′-cyclophosphate de formule I selon la revendication 1 pour la préparation de roboflavine-5′-phosphate ou de son sel monosodique par hydrolyse, isomérisation et, au besoin, neutralisation partielle.

6. Procédé selon la revendication 5, caractérisé en ce que, pour la préparation de riboflavine-5′-phosphate ou de son sel monosodique,
   a) on hydrolyse le chlorure d'acide riboflavine-4′,5′-cyclophosphorique de formule I obtenu en riboflavine-4′,5′-phosphate,
   b) on isomérise celui-ci en riboflavine-5′-phosphate et
   c) on fait au besoin réagir ce dernier avec de l'hydroxyde de sodium pour obtenir son sel monosodique.

7. Procédé selon la revendication 5, caractérisé en ce que, pour le préparation de roboflavine-5′-phosphate
   a) on additionne rapidement le mélange réactionnel obtenu, contenant du chlorure d'acide riboflavine-4′,5′-phosphorique de formule I, de 30 à 50 moles d'eau par mole de chlorure de riboflavine-4′,5′-cyclophosphate, la température s'élevant alors à plus de 90°C et du riboflavine-4′,5′-phosphate étant formé par hydrolyse,
   b) on maintient le mélange réactionnel pendant 5 à 15 mn encore à des températures de 80 à 100°C par injection de vapeur, le riboflavine-4′,5′-phosphate formé étant ainsi isomérisé en riboflavine-5′-phosphate, et
   on interrompt la réaction d'isomérisation par addition subséquente de 68 à 100 moles d'eau au mélange réactionnel et par le refroidissement qui en résulte.

8. Procédé selon la revendication 5 caractérisé en ce que, pour la préparation du sel monosodique de riboflavine-5′-phosphate,
   a) on additionne rapidement le mélange réactionnel obtenu, contenant du chlorure d'acide riboflavine-4′,5′-phosphorique de formule I, de 30 à 35 moles d'eau par mole de chlorure de riboflavine-4′,5′-cyclophosphate la température s'élevant alors à plus de 90°C et du riboflavine-4′,5′-phosphate étant formé par hydrolyse,
   b) on maintien le mélange réactionnel pendant environ 5 à 15 mn encore à des températures de 80 à 100°C par injection de vapeur, le riboflavine-4′,5′-phosphate formé étant isomérisé en riboflavine-5′-phosphate
c) on interrompt la réaction d'isomérisation par addition subséquent de 68 à 100 moles d'eau au mélange réactionnel et par le refroidissement qui en résulte et
d) on règle le pH du mélange réactionnel à une valeur de 5,5 à 6 avec de l'hydroxyde de sodium.

**9.** Procédé de préparation de riboflavine-4′,5′-phosphate ou de son sel monosodique, caractérisé en ce que
a) on introduit du chlorure de riboflavine-4′,5′-cyclophosphate cristallin dans une quantité suffisante pour sa dissolution d'eau chauffée à 80-95°C,
b) on maintient le mélange réactionnel pendant 5 à 15 mn encore à des températures de 80 à 100°C par injection de vapeur,
c) on ajoute ensuite de 68 à 100 moles d'eau et on isole le riboflavine-5′-phosphate qui se sépare à l'état cristallin ou, au besoin,
d) pour la préparation du sel monosodique, on règle le pH du mélange réactionnel à une valeur de 5,5 à 6 avec de l'hydroxyde de sodium et on isole le sel monosodique du riboflavine-5′-phosphate qui se sépare à l'état cristallin.

**10.** Procédé de préparation de riboflavine-5′-phosphate pur ou de son sel monosodique, caractérisé en ce que
A) on fait réagir un sel alcalin de riboflavine, dans un solvant aprotique approprié, à des températures de 20 à 50°C, avec 1,2 à 3 moles d'oxychlorure de phosphore par mole du sel alcalin,
B) on additionne rapidement le mélange réactionnel obtenu, contenant le nouveau chlorure de riboflavine-4′,5′-cyclophosphate, de 30 à 50 moles d'eau par mole de chlorure d'ester, la température s'élevant alors à plus de 90°C,
C) on maintient le mélange réactionnel pendant 5 à 15 mn encore à des températures de 80 à 100°C par injection de vapeur,
D) on ajoute ensuite 68 à 100 mole d'eau au mélange réactionnel et on isole le riboflavine-5′-phosphate qui se sépare à l'état cristallin ou, au besoin,
E) on règle le pH du mélange réactionnel obtenu en D), à des températures de 20 à 50°C, à un valeur comprise entre 5,5 et 6 avec NaOH et on isole le sel monosodique de riboflavine-5′-phosphate qui sépare à l'état cristallin.